# EUROPEAN PATENT APPLICATION

(11) **EP 3 991 723 A1**
(43) Date of publication of application: **04.05.2022**
(21) Application number: 20831494.8
(22) Date of filing: 03.06.2020
(51) Int. Cl.: A61K 9/16, A61K 9/20, A61K 9/28, A61K 36/287, A61P 19/00, A23L 33/105

(54) **PHARMACEUTICAL COMPOSITION WITH IMPROVED STABILITY COMPRISING NATURAL PRODUCT EXTRACT**

(30) Priority: 26.06.2019 KR 20190076519
(71) Applicant: Green Cross WellBeing Corporation, Seongnam-si, Gyeonggi-do 13595 (KR)
(72) Inventor: LIM, Nak Hyun, Seongnam-si, Gyeonggi-do 13595 (KR); LEE, Jong Hun, Seongnam-si, Gyeonggi-do 13595 (KR); LIM, Su Hwan, Seongnam-si, Gyeonggi-do 13595 (KR); KIM, Jeom Yong, Seongnam-si, Gyeonggi-do 13595 (KR); PARK, Sun Kyu, Seongnam-si, Gyeonggi-do 13595 (KR)
(74) Representative: dompatent von Kreisler Selting Werner - Partnerschaft von Patent- und Rechtsanwälten mbB
(86) International application number: PCT/KR2020/007226
(87) International publication number: WO 2020/262838

(57) **Abstract**

Disclosed is a pharmaceutical composition with improved stability comprising a natural product extract, in which the content of an active ingredient in the extract is stably maintained through the addition of only a small number of additives to a whole plant extract of Chrysanthemum zawadskii or a closely related plant of the same genus, which is effective in the prevention and treatment of osteoarthropathy and the like but is vulnerable to moisture, the pharmaceutical composition being capable of securing various stabilities against moisture and the like when formulated into a drug. The pharmaceutical composition with improved stability comprising a natural product extract comprises Chrysanthemum zawadskii extract granules obtained by mixing a Chrysanthemum zawadskii extract and a dextrin-based compound and granulating the mixture.

## Description

### [Technical Field]

The present application claims the benefit of priority based on Korean Patent Application No. 10-2019-0076519 filed on June 26, 2019, the entire contents of which are incorporated herein by reference.

The present invention relates to a pharmaceutical composition with improved stability comprising a natural product extract, and more particularly, to a pharmaceutical composition with improved stability comprising a natural product extract, in which the content of an active ingredient in the extract is stably maintained through the addition of only a small number of additives to a whole plant extract of Chrysanthemum zawadskii or a closely related plant of the same genus, which is effective in the prevention and treatment of osteoarthropathy and the like but is vulnerable to moisture, the pharmaceutical composition being capable of securing various stabilities against moisture and the like when formulated into a drug.

### [Background Art]

Arthritis is a disease in which joints are inflamed and damaged by various causes, and the main symptoms are joint damage caused by inflammation in the joint area and accompanied pain. However, all joint pains cannot be called arthritis, and swelling or fever must be accompanied to be called arthritis. Such arthritis is classified into at least 100 types, and among them, osteoarthritis (OA) and rheumatoid arthritis (RA) are the most frequently occurring.

The osteoarthritis (OA) is arthritis caused by degenerative changes in cartilage and marginal bone in synovial joints, and is the most common joint disease that has severe pain and movement disorders in the knee joint and hip joint, which receive a lot of weight and causes even joint deformation if untreated for a long time. In particular, the osteoarthritis (OA) is a degenerative disease commonly occurring in older people with reduced metabolism and cell regeneration ability, and fundamental treatment is almost impossible, and surgery, drug therapy, and physical therapy are being performed. However, except for some adjuvant therapies, many side effects are accompanied, and the development of new therapeutic agents is urgently needed.

The rheumatoid arthritis (RA) is an autoimmune disease in which a non-bacterial inflammatory response is chronically displayed in the synovial membrane as a systemic inflammatory disease, and as a result, the synovial membrane is proliferated and the amount of synovial fluid is increased, resulting in joint swelling and pain. In addition, the rheumatoid arthritis (RA) affects not only the joints but also various parts of the body, which is also called a rheumatoid disease. With regard to these causes, exact factors that cause the immune response are not yet known, but it is known that the autoimmune response plays an important role in the chronicity and progression of this disease. In other words, the rheumatoid arthritis (RA) is a continuous autoimmune response in which T cells play an important role in addition to the local release of inflammatory mediators and cytokines, resulting in destroying the joints, and main symptoms are fatigue, weakness, pain, and the like, and accompanied by fever and weakness as the arthritis progresses. According to research, about 1% of the population suffers from this disease, and this disease may appear at various ages, but is particularly common in those in the 30s and 40s.

In addition, osteoporosis is a metabolic disease in which bone integrity and strength are weakened due to qualitative changes in the bone microstructure to increase the risk of fractures in the spine, femur, and radius, and with the improvement of living standards and medical development, as the average life expectancy is getting longer, the elderly population is also increasing, and accordingly, osteoporosis is also continuously increasing. Normal bone is an active metabolic organ in which bone resorption by osteoclasts, the formation of new bone matrix by osteoblasts, and subsequent mineralization processes occur continuously and repeatedly, and the average bone metabolism has a cycle of 3 to 4 months. From birth to adulthood after growing up, since bone production is greater than bone resorption, the bone mass gradually increases, and the maximum bone mass is formed in the 30s. Thereafter, as the age increases, resorption increases rather than bone formation, leading to a decrease in bone density of 0.4 to 2% per year. In particular, in the case of women, as estrogen decreases after menopause, bone resorption by osteoclasts increases significantly compared to before menopause, resulting in rapid bone loss of 1 to 5% per year.

In modern times, although arthritis is a common disease that has become wider and lowered in the age group due to a rapid increase in obese patients due to changes in eating habits and development of transportation, the pathogenesis of arthritis is not yet clear, and thus, there is also a shortage of selective therapeutic drugs. Accordingly, the therapeutic purpose of arthritis is to reduce joint inflammation and pain, and to prevent joint deformation. Since there is no fundamental treatment for arthritis, physical exercise therapy is performed as a basic therapy, and appropriate non-steroidal anti-inflammatory drugs (NSAIDs) are used to relieve inflammation, and if inflammation is severe, gold salt therapy or penicillamine therapy has been performed. If the method is ineffective, steroids are used, and in the case of intractability, immunosuppressive drugs are used, and if the arthritis is converted to arthritis deformans, surgery is performed.

Non-steroidal anti-inflammatory drugs (NSAIDs) used for anti-inflammatory and analgesic effects for arthritis mainly inhibit cyclooxygenase to suppress the production of prostaglandin, which is involved in the inflammatory reaction, thereby exhibiting anti-inflammatory effects, and among the drugs, indomethacin and phenylbutazone have a strong anti-inflammatory effect compared to other NSAIDs. Indomethacin, as one of drugs of most strongly inhibiting the production of prostaglandin causing inflammation, is well absorbed by oral administration, but this drug has severe toxicity that dizziness, delirium and, rarely, psychosis with hallucinations have been reported, in addition to side effects of the general gastrointestinal tract shown in NSAIDs, and has many side effects, such as stopping medication in 33% of patients at high doses. Phenylbutazone has been also effectively used in the treatment of rheumatoid arthritis, ankylosing spondylitis, and acute gouty arthritis compared to other NSAIDs at the beginning of its use, but has been not currently used because of severe toxicity including hematologic abnormalities that cause granulocytopenia, aplastic anemia, or the like.

Compared to these NSAIDs, steroids are the fastest method available to patients, and are known as drugs that have rapid and dramatic anti-inflammatory effects and give patients pleasure. However, as widely known, this drug weakens the resistance to bacterial infection, and is very serious in toxicity, such as exacerbation of diabetes, Cushing's syndrome, adrenal insufficiency, mental dysfunction, etc. and difficult to stop the treatment after starting, and thus, it is known that this drug should be avoided if possible, such as requiring caution in use. As such, since synthetic anti-inflammatory agents are often accompanied by various side effects, as described above, there is a steady demand for the development of anti-inflammatory agents with strong efficacy and relatively few side effects. In this regard, in the case of herbal preparations and natural product preparations that have been used in ordinary people for a long time and have abundant clinical experience and are highly evaluated in terms of safety, there is an advantage of having a high possibility of being developed as materials for treating arthritis.

Among them, Chrysanthemum zawadskii var. latilobum Kitamura belongs to the Asteraceae and is wild throughout Korea, and includes Chrysanthemum zawadskii var. alpinum, Chrysanthemum zawadskii Herbich, Chrysanthemum zawadskii var. tenuisectum, Chrysanthemum zawadskii var. leiophyllum T. LEE., etc. as the same genus. In the ordinary people, Chrysanthemum zawadskii has been mainly used to be effective for cold constitution, dysmenorrhea, and menstrual irregularities in women's diseases, and there is also a patent document on a composition for preventing and treating osteoarthropathy including osteoporosis and arthritis using a Chrysanthemum zawadskii extract.

That is, Korean Patent Registration No. 10-1183573 relates to 'a composition for preventing and treating osteoarthropathy containing a Chrysanthemum zawadskii extract as an active ingredient'. In addition, there is disclosed a content that the Chrysanthemum zawadskii extract reduces joint inflammation and inhibits the expression of inflammatory mediators and cytokines, and furthermore, has not only an excellent preventive or therapeutic effect on arthritis in an animal model of collagen-induced arthritis disease, but also has a mechanism that the differentiation of osteoclasts that play an important role in osteoporosis as well as arthritis and bone resorption by osteoclasts, which cause a problem in osteoporosis, are inhibited by the Chrysanthemum zawadskii extract, thereby applying the extract to prevention or treatment of osteoarthropathy, including arthritis and osteoporosis.

However, most of the natural extracts including the document are mixed with additives such as solubilizers and disintegrants during pharmaceutical formulation. In this case, there is a problem in that stability is lowered at the time of mixing with natural extracts and additives and a subsequent storage of a finished product, such as reducing the content of active ingredients contained in the natural extracts. In addition, the natural extract itself has very low moisture content stability, so that there are also problems in that a moisture-proof coating treatment is required on the surface of the drug, and accordingly, the total weight of the finished product is increased.

Therefore, the development of a novel pharmaceutical composition is urgently required, so that even if only a minimal additive is added to the natural extract, that is, the Chrysanthemum zawadskii extract, the content of the active ingredients in the extract is stably maintained, and even if the moisture-proof coating treatment is not applied on the surface of the drug or minimized, the moisture content of the extract may be stably maintained.

### [Disclosure]

### [Technical Problem]

An object of the present invention is to provide a pharmaceutical composition with improved stability comprising a natural product extract, in which the content of an active ingredient in the extract is stably maintained through the addition of only a small number of additives to a whole plant extract of Chrysanthemum zawadskii or a closely related plant of the same genus, which is effective in the prevention and treatment of osteoarthropathy or the like and the like, but is vulnerable to moisture.

Another object of the present invention is to provide a pharmaceutical composition with improved stability comprising a natural product extract, capable of securing stability against moisture when a whole plant extract of Chrysanthemum zawadskii or a closely related plant of the same genus is formulated into a drug.

Yet another object of the present invention is to provide a pharmaceutical composition with improved stability comprising a natural product extract, capable of securing stability and improving mixability at the time of pharmaceutical formulation by mixing and granulating a dextrin-based compound with a viscous liquid whole plant extract of Chrysanthemum zawadskii or a closely related plant of the same genus.

### [Technical Solution]

An aspect of the present invention provides a pharmaceutical composition comprising Chrysanthemum zawadskii extract obtained by mixing a Chrysanthemum zawadskii extract and a dextrin-based compound and granulating the mixture.

Another aspect of the present invention provides a food composition comprising Chrysanthemum zawadskii extract granules obtained by mixing a Chrysanthemum zawadskii extract and a dextrin-based compound and granulating the mixture.

### [Advantageous Effects]

According to the present invention, the pharmaceutical composition with improved stability comprising the natural product extract has an advantage of stably maintaining the content of active ingredients in the extract through the addition of only a small number of additives to a whole plant extract of Chrysanthemum zawadskii or a closely related plant of the same genus, which is effective in the prevention and treatment of osteoarthropathy or the like and the like, but is vulnerable to moisture. Further, there are advantages of not only securing stability against moisture when a whole plant extract of Chrysanthemum zawadskii or a closely related plant of the same genus is formulated into a drug, but also securing stability and improving mixability at the time of pharmaceutical formulation by mixing and granulating a dextrin-based compound with a viscous liquid whole plant extract of Chrysanthemum zawadskii or a closely related plant of the same genus.

### [Best Mode]

Hereinafter, the present invention will be described in detail.

A pharmaceutical composition with improved stability comprising a natural product extract according to the present invention comprises Chrysanthemum zawadskii extract granules obtained by mixing a Chrysanthemum zawadskii extract with a dextrin-based compound and granulating the mixture.

The Chrysanthemum zawadskii is a resource plant native to Korea and refers to Chrysanthemum zawadskii var. latilobum Kitamura, and specifically, is characterized by Chrysanthemu zawadskii (or sibirium) var. latilobum, or at least one selected from the group consisting of Chrysanthemum zawadskii var. tenuisectum, Chrysanthemum zawadskii subsp. coreanum, Chrysanthemum zawadskii subsp. Naktongense, and Chrysanthemum zawadskii var. alpinum, and there are no special restrictions on the parts to be used, such as flowers, leaves, stems and roots.

In the present invention, the 'extract' means an active ingredient that may be separated from a natural product, and is prepared by an extraction process using water, organic solvents or mixed solvents thereof, and includes extracts of water, organic solvents or mixed solvents thereof, dry powders thereof, or any form formulated using the same. In addition, the extract includes fractions of the extract that has been subjected to the extraction process.

In addition, the solvent used in the preparation of the extract may be water, organic solvents, or mixed solvents thereof, specifically, water, organic solvents such as polar solvents such as low-cost alcohols having 1 to 4 carbon atoms such as methanol and ethanol, and ethyl acetate, and non-polar solvents such as hexane or dichloromethane, or mixed solvents thereof. In addition, the solvent may be preferably water, alcohols having 1 to 4 carbon atoms or mixed solvents thereof, more preferably water or a mixed solvent of water or ethanol, but is not limited thereto. In addition, in the present invention, the Chrysanthemum zawadskii extract is preferably extracted by an additive-mixed boiling water extraction method or an additive-mixed solvent extraction method, but is not limited thereto, and the method is not particularly limited so long as the method may be any method capable of extracting active ingredients from a natural product, such as a general natural produce extraction method and the like.

For example, the preparation method of the Chrysanthemum zawadskii extract by the additive-mixed boiling water extraction method may comprises steps of 1) washing Chrysanthemum zawadskii with distilled water and finely grinding Chrysanthemum zawadskii with a grinder, 2) heating the ground Chrysanthemum zawadskii in boiling water to obtain a viscous liquid Chrysanthemum zawadskii extract, 3) adding a dextrin-based compound to the obtained Chrysanthemum zawadskii extract and then grinding the mixture to prepare a powder-granulated Chrysanthemum zawadskii extract.

The largest feature of the additive-mixed boiling water extraction method is to add the dextrin-based compound to the Chrysanthemum zawadskii extract at a predetermined ratio, and a general natural extract has a viscous liquid form and thus, it is preferred to mix additives to improve storage stability and make it easier to form formulations such as tablets or capsules. Accordingly, the present applicants added and mixed the dextrin-based compound to the Chrysanthemum zawadskii extract obtained as described above and then powder-granulated the Chrysanthemum zawadskii extract through a drying and grinding process, and as a result, it is possible to maintain the content of active ingredients (or effective ingredients) such as Linarin contained in the Chrysanthemum zawadskii extract without changes even after a considerable period of time has elapsed.

On the other hand, a natural extract has a moisture property, and accordingly, it is preferred to maintain a predetermined level of moisture content, but there have been difficulties therein so far. However, in the present invention, by mixing the Chrysanthemum zawadskii extract with the dextrin-based compound as described above, the moisture content was maintained in an appropriate state without significantly increasing or decreasing.

The dextrin-based compound basically serves to make a Chrysanthemum zawadskii extract having a high viscosity (for example, in the form of starch syrup) into a form of granules, and may be 50 to 70 wt%, preferably 55 to 65 wt%, more preferably about 60 wt% based on the total weight of the Chrysanthemum zawadskii extract granules. On the other hand, in the present specification, the term 'granules' may include fine powder, and specifically, may be in the form of a mixture of granular particles and fine powder particles, or may be formed by agglomerating fine powder particles. In this case, the size of the granular particles may be particle sizes of pharmaceuticals generally manufactured in the art, and the size of the particles is not particularly limited as long as the purpose of the present invention of improving stability may be achieved.

If the content of the dextrin-based compound is less than 50 wt% based on the total weight of the Chrysanthemum zawadskii extract granules, raw materials are made in the form of granules or powders that are more humid (containing a lot of moisture), and when a finished product is prepared using the raw materials, the binding properties of the raw materials are increased and the anti-deliquescent effect of the natural product extract is reduced, so that the disintegration time may be delayed. In addition, when the content of the dextrin-based compound exceeds 70 wt% based on the total weight of the Chrysanthemum zawadskii extract granules, the ratio of the Chrysanthemum zawadskii extract is relatively reduced, so that a large amount of mixture (Chrysanthemum zawadskii extract + dextrin-based compound) is used in a formulation test, and as a result, when the finished product is prepared, there is a risk of problems that tabletability is lowered and a disintegration time or the like is changed.

Such a dextrin-based compound may include maltodextrin, cyclodextrin, a dextrin-based compound having similar or identical physical properties and characteristics, and mixtures thereof, and it is preferred to use maltodextrin in terms of economics.

In addition, in step 1), it is preferred to add 1.0 L of water to 100 g of Chrysanthemum zawadskii, and in step 2), it is preferred to heat and extract Chrysanthemum zawadskii in boiling water at 100 to 120°C for 2 to 3 hours, and at this time, it is preferred to install a cooler to maintain a predetermined temperature and prevent loss to steam.

In addition, the preparation method of the Chrysanthemum zawadskii extract through the additive-mixed solvent extraction method may comprise steps of 1) immersing Chrysanthemum zawadskii in low alcohol and extracting the immersed Chrysanthemum zawadskii with an ultrasonic extractor to obtain a Chrysanthemum zawadskii extract, and 2) adding a dextrin-based compound to the obtained Chrysanthemum zawadskii extract and then grinding the mixture to prepare a powder-granulated Chrysanthemum zawadskii extract.

At this time, it is preferred to obtain an alcohol extract of Chrysanthemum zawadskii, which exhibits a prevention and treatment effect of arthritis and osteoporosis by immersing Chrysanthemum zawadskii in low alcohol and repeatedly extracting the immersed Chrysanthemum zawadskii. More preferably, in step 1), it is preferred to use ethanol at a concentration of 10 to 95% as the low alcohol, and it is preferred to obtain an extract by treating an ultrasonic extractor for 2 to 3 weeks. In addition, since the addition amount and the like of the dextrin-based compound are the same as those described in the additive-mixed boiling water extraction method, a detailed description thereof will be omitted.

The pharmaceutical composition may further comprise crystalline cellulose in addition to the aforementioned Chrysanthemum zawadskii extract granules (Chrysanthemum zawadskii extract and dextrin-based compound). The crystalline cellulose is a component that may be used together with a lubricant to be described below to secure stability against moisture, and accordingly, when medicating the Chrysanthemum zawadskii extract according to the present invention, tablet coating corresponds to a general coating, not moisture-proof coating. The crystalline cellulose may be used in the content of 50 to 70 wt%, preferably 50 to 65 wt%, more preferably 50 wt% or more and less than 60 wt% based on the total weight of the pharmaceutical composition of the present invention. When the total weight of an initial finished composition is rather high in consideration of the size and the like of the tablets, the contents or weights of the crystalline cellulose and the lubricant to be described below may be lowered. That is, the additive ratio may be adjusted in consideration of the size of the tablets.

If the content of crystalline cellulose is less than 50 wt% based on the total weight of the pharmaceutical composition of the present invention, the disintegration time may be delayed compared to existing compositions, and in addition, a deliquescent preventing effect may be lowered to exhibit a change in stability due to an increased moisture content. In addition, when the content of crystalline cellulose exceeds 70 wt% based on the total weight of the pharmaceutical composition of the present invention, the ratio of the active ingredient in the mixture of the same weight is relatively high due to the increase in the content of the crystalline cellulose, and the tablet size may vary because the tablets need to be made with an excess of the mixture. In addition, when the tablet size is maintained in this state, it is necessary to increase a tableting pressure, and as a result, the hardness is increased, which may affect disintegration. In addition, during tableting, the surface is not smooth, the binding force is reduced, and friability is increased, and as a result, tableting and coating problems may occur.

The pharmaceutical composition with improved stability comprising the natural product extract according to the present invention may further comprise a pharmaceutically acceptable excipient, but does not comprise a solubilizer and a disintegrant.

The excipient may be a lubricant, and the lubricant may include lubricants commonly used in the art, such as silicon dioxide, magnesium stearate, and mixtures thereof, but it is preferable to apply silicon dioxide and magnesium stearate together. In addition, the lubricant may be used in the content of 2 to 4 wt% based on the total weight of the pharmaceutical composition of the present invention.

On the other hand, the pharmaceutical composition with improved stability comprising the natural product extract according to the present invention may be formulated in the form selected from the group consisting of tablets, pills, powders, granules, capsules, suspensions, oral liquids, emulsions, syrups, and freeze-dried preparations, but may be most preferably formulated in the form of tablets in consideration of the purpose of the present invention.

When the pharmaceutical composition is formulated in the form of tablets, the tablet may include a coating layer, and the coating layer may include only an aqueous coating agent. That is, as described above, as the present invention secures stability against moisture by applying crystalline cellulose, a lubricant, etc. at specific contents, existing moisture-proof coating agents are not included (that is, the pharmaceutical composition has a moisture-proof effect even without including a moisture-proof coating agent, and the moisture-proof effect may be expressed by any one or more of a dextrin-based compound, crystalline cellulose, and a lubricant).

The aqueous coating agent may include, for example, hydroxypropylmethylcellulose (HPMC), glycerin fatty acid ester, titanium dioxide, loc color, and materials having similar or identical physical properties and characteristics, and the HPMC may be replaced with methylcellulose, ethylcellulose, hydroxypropylcellulose, or cellulose-based compounds having properties similar thereto. In addition, the aqueous coating agent may further include any one or more of a solubilizer, a plasticizer, a binder, a lubricant, a wetting agent, a sweetener, a flavoring agent, an emulsifier, a suspension, and a diluent, if necessary.

The pharmaceutical composition of the present invention comprising the above ingredients has various advantages in addition to the aforementioned advantages. First, the pharmaceutical composition of the present invention may reduce the content of the drug without using a solubilizer, a disintegrant, and a moisture-proof coating agent unlike the related art. In addition, in the case of general tablets, the hardness increases as the moisture content decreases, and accordingly, the disintegration time also tends to increase, but in the case of tablets using the pharmaceutical composition of the present invention, as the moisture content is maintained, the hardness does not increase, and accordingly, there is an advantage that a time required for disintegration is constant within 30 minutes even without using a disintegrant, etc (unaffected by storage temperature and duration).

Meanwhile, in the present invention, the term "osteoarthropathy" refers to all types of bone diseases, and among them, includes arthritis and osteoporosis. The osteoarthropathy is characterized to inhibit the inflammatory response of arthritis, differentiation of osteoclasts, and bone resorption, but is not limited thereto.

In addition, the composition for preventing or treating osteoarthropathy of the present invention may be prepared as a food, particularly, functional food composition, and may comprise Chrysanthemum zawadskii extract granules obtained by mixing a Chrysanthemum zawadskii extract and a dextrin-based compound and granulating the mixture, and the detailed description of the dextrin-based compound follows those described in the pharmaceutical composition.

The food composition may include ingredients that are commonly added in addition to the Chrysanthemum zawadskii extract granules, and may include common ingredients, such as proteins, carbohydrates, fats, nutrients and seasonings. In addition, the food composition may further comprise a food acceptable supplement additive, and the food composition may be formulated in the form of pills, powders, granules, infusions, tablets, capsules or beverages. In addition, in the present invention, the food means natural products or processed products containing one or more nutrients, preferably states that can be directly ingested through a processing process, more preferably foods, food additives, health functional foods, beverages, etc.

In addition, it is specified that a dose level for a specific patient of the composition according to the present invention may be varied according to the weight, age, and sex of a patient, health condition, diet, administration time, administration method, excretion rate, the severity of disease, etc.

Hereinafter, preferred Examples will be proposed to help in understanding of the present invention. However, the following Examples are just illustrative of the present invention, and it will be apparent to those skilled in the art that various changes and modifications can be made within the scope and the technical idea of the present invention and it will be natural that these changes and modifications cover the appended claims.

### [Example 1] Preparation of Chrysanthemum zawadskii extract granules

First, Chrysanthemum zawadskii and a whole plant of the same genus were washed with distilled water and finely ground with a grinder, and then heated in boiling water of 100 to 120°C for 2 to 3 hours to obtain a viscous liquid Chrysanthemum zawadskii extract (when heating, a cooler was installed to maintain a predetermined temperature and prevent loss of steam), and the obtained Chrysanthemum zawadskii extract was added with maltodextrin at about 60 wt% and then ground and powder-granulated to prepare Chrysanthemum zawadskii extract granules.

### [Test Example 1] Evaluation of content of Linarin ingredient included in Chrysanthemum zawadskii extract

In order to confirm the stability according to the mixing of a Chrysanthemum zawadskii extract and maltodextrin, the Chrysanthemum zawadskii extract granules (Chrysanthemum zawadskii extract 40 wt% + maltodextrin 60 wt%) prepared in Example 1 were left at 25°C, 35°C, and 40°C for 9 months, respectively, and then the content of a Linarin ingredient was evaluated over time, and the result thereof was shown in Table 1 below.

**[Table 1]**

| | Month | Linarin content (%) | | |
|---|---|---|---|---|
| | | 25°C | 35°C | 40°C |
| Chrysanthemum zawadskii extract granules (Chrysanthemum zawadskii extract 40 wt% + maltodextrin 60 wt%) | 0 | 0.977 | 0.977 | 0.977 |
| | 1 | 0.977 | 0.977 | 0.973 |
| | 2 | 0.968 | 0.953 | 0.971 |
| | 3 | 0.958 | 0.973 | 0.968 |
| | 4 | 0.968 | 0.971 | 0.965 |
| | 5 | 0.968 | 0.966 | 0.959 |
| | 6 | 0.966 | 0.962 | 0.955 |
| | 7 | 0.948 | 0.961 | 0.953 |
| | 8 | 0.954 | 0.956 | 0.950 |
| | 9 | 0.952 | 0.952 | 0.951 |

As a result of evaluating the content of the Linarin ingredient through the method as described above, as shown in Table 1 above, in all of a storage condition (25°C), an intermediate acceleration condition (35°C) and an acceleration condition (40°C), it was confirmed that the Linarin content after 9 months was maintained at 97% or more compared to initial measurement (i.e., it was confirmed that there was no significant difference). From this, it could be seen that there was no problem in compatibility between the Chrysanthemum zawadskii extract and maltodextrin.

### [Test Example 2] Evaluation of moisture content of Chrysanthemum zawadskii extract

In order to confirm another stability according to the mixing of a Chrysanthemum zawadskii extract and maltodextrin, the Chrysanthemum zawadskii extract granules (Chrysanthemum zawadskii extract 40 wt% + maltodextrin 60 wt%) prepared in Example 1 were left at 25°C, 35°C, and 40°C for 9 months, respectively, and then the moisture content was evaluated over time, and the result thereof was shown in Table 2 below.

**[Table 2]**

| | Month | Moisture content (%) | | |
|---|---|---|---|---|
| | | 25°C | 35°C | 40°C |
| Chrysanthemum zawadskii extract granules (Chrysanthemum zawadskii extract 40 wt% + maltodextrin 60 wt%) | 0 | 2.85 | 2.85 | 2.85 |
| | 1 | 2.74 | 2.78 | 2.91 |
| | 2 | 3.58 | 2.93 | 2.94 |
| | 3 | 2.87 | 2.85 | 2.87 |
| | 4 | 2.63 | 2.71 | 2.76 |
| | 5 | 2.76 | 2.83 | 2.92 |
| | 6 | 2.55 | 2.62 | 2.63 |
| | 7 | 2.53 | 2.63 | 2.53 |
| | 8 | 2.48 | 2.39 | 2.32 |
| | 9 | 2.36 | 2.32 | 2.30 |

As a result of evaluating the moisture content through the method as described above, as shown in Table 2 above, in all of a storage condition (25°C), an intermediate acceleration condition (35°C) and an acceleration condition (40°C), it was confirmed that the moisture content for 9 months was maintained at about 2 to 3% steadily from the initial measurement. That is, it could be seen that there was no decrease or increase in moisture content of the Chrysanthemum zawadskii extract according to the mixing with maltodextrin, and based on this, the Chrysanthemum zawadskii extract and maltodextrin were mixed in an appropriate ratio. In addition, it was confirmed that in the case of the natural extract, it was important to maintain a certain level of moisture content by exhibiting moisture properties, which was also satisfactory.

### [Example 2] Preparation of pharmaceutical composition comprising Chrysanthemum zawadskii extract (unfinished product)

According to compositions of Table 3 below, a pharmaceutical composition (800 mg) containing a Chrysanthemum zawadskii extract, which secured moisture stability while minimizing additives, was prepared, and specific characteristics and properties of the composition were as follows.
- Composition for the purpose of securing stability for a certain period of time and completing disintegration within 30 minutes even after excluding a solubilizer and a disintegrant such as sodium bicarbonate.
- Using crystalline cellulose at 50% or more based on total weight in order to secure stability of a natural extract, which was vulnerable to moisture.
- Without using moisture-proof coating base to minimize changes in Linarin and moisture content.
- Composition that may lower the total weight while maintaining stability when considering a tablet size, etc. by setting the total weight of an initial finished composition to 800 mg.

**[Table 3]**

| | Ingredient name | Weight (mg) | Ratio (%) |
|---|---|---|---|
| Tablet | Chrysanthemum zawadskii extract granules | 250.00 | 31.25 |
| | Crystal cellulose | 502.00 | 62.75 |
| | Silicon dioxide (lubricant) | 14.40 | 1.80 |
| | Magnesium stearate (lubricant) | 9.60 | 1.20 |
| Coating | HPMC | 16.55 | 2.07 |
| | Glycerin fatty acid ester | 4.97 | 0.62 |
| | Titanium dioxide | 1.66 | 0.21 |
| | Lac color | 0.82 | 0.11 |
| Total | | 800 | 100 |

### [Example 3] Preparation of pharmaceutical composition comprising Chrysanthemum zawadskii extract (finished product)

According to compositions of Table 4 below, even while maintaining the characteristics and properties of the pharmaceutical composition (unfinished) prepared in Example 2 as they are, the total weight was lowered while maintaining stability to prepare a pharmaceutical composition (600 mg) containing a Chrysanthemum zawadskii extract. In other words, a composition was prepared to secure the stability of the raw material by mainly lowering the ratio or content of crystalline cellulose and a coating base.

**[Table 4]**

| | Ingredient name | Weight (mg) | Ratio (%) |
|---|---|---|---|
| Tablet | Chrysanthemum zawadskii extract granules | 250.00 | 41.67 |
| | Crystal cellulose | 314.00 | 52.33 |
| | Silicon dioxide (lubricant) | 10.80 | 1.80 |
| | Magnesium stearate (lubricant) | 7.20 | 1.20 |
| Coating | HPMC | 12.41 | 2.07 |
| | Glycerin fatty acid ester | 3.73 | 0.62 |
| | Titanium dioxide | 1.24 | 0.21 |
| | Lac color | 0.62 | 0.10 |
| Total | 600 | 100 | |

### [Test Example 3] Evaluation of content of Linarin ingredient included in finished product

In order to confirm stability of the pharmaceutical composition (600 mg, finished product) including the Chrysanthemum zawadskii extract prepared in Example 3, a finished product (600 mg) was left for 9 months at 25°C, 35°C, and 40°C, respectively, and then the content of the Linarin ingredient was evaluated over time, and the result thereof was shown in Table 5 below.

**[Table 5]**

| | Month | Linarin content (%) | | |
|---|---|---|---|---|
| | | 25°C | 35°C | 40°C |
| Finished product, 600 mg | 0 | 0.375 | 0.375 | 0.375 |
| | 1 | 0.359 | 0.361 | 0.374 |
| | 2 | 0.367 | 0.379 | 0.366 |
| | 3 | 0.354 | 0.368 | 0.366 |
| | 4 | 0.362 | 0.363 | 0.364 |
| | 5 | 0.370 | 0.359 | 0.363 |
| | 6 | 0.363 | 0.368 | 0.363 |
| | 7 | 0.368 | 0.374 | 0.363 |
| | 8 | 0.367 | 0.365 | 0.361 |
| | 9 | 0.366 | 0.367 | 0.356 |

As a result of evaluating the content of the Linarin ingredient included in the finished product through the method as described above, as shown in Table 5 above, in all of a storage condition (25°C), an intermediate acceleration condition (35°C) and an acceleration condition (40°C), it was confirmed that the Linarin content did not show a significant change for 9 months (that is, it was confirmed that there was no significant increase or decrease in content), and considering an error occurred during the test, it could be seen that there was actually no change in the content of Linarin in the finished product. In other words, the stability of the finished product was confirmed under various conditions.

### [Test Example 4] Evaluation of moisture content in finished product

In order to confirm another stability of the pharmaceutical composition (600 mg, finished product) including the Chrysanthemum zawadskii extract prepared in Example 3, a finished product (600 mg) was left for 9 months at 25°C, 35°C, and 40°C, respectively, and then the moisture content was evaluated over time, and the result thereof was shown in Table 6 below.

**[Table 6]**

| | Month | Moisture content (%) | | |
|---|---|---|---|---|
| | | 25°C | 35°C | 40°C |
| Finished product, 600 mg | 0 | 3.38 | 3.38 | 3.38 |
| | 1 | 3.29 | 3.42 | 3.43 |
| | 2 | 3.75 | 3.39 | 3.00 |
| | 3 | 3.33 | 3.30 | 3.43 |
| | 4 | 2.75 | 2.76 | 2.58 |
| | 5 | 3.48 | 3.29 | 3.22 |
| | 6 | 2.74 | 2.55 | 2.65 |
| | 7 | 2.83 | 2.79 | 2.56 |
| | 8 | 2.07 | 2.00 | 2.24 |
| | 9 | 1.81 | 1.82 | 1.90 |

As a result of evaluating the moisture content contained in the finished product through the method described above, as shown in Table 6 above, even though the additive was added, it could be seen that the moisture content tended to decrease in all of a storage condition (25°C), an intermediate acceleration condition (35°C), and an acceleration condition (40°C) as the period elapsed. That is, since there was no change in evaluation of the moisture content of the Chrysanthemum zawadskii extract granules (Chrysanthemum zawadskii extract + maltodextrin) (see Test Example 2 above), the above results were due to the fact that an excipient included in a final product absorbed moisture, and the absorbed moisture was vaporized. Through these results and the evaluation of the Linarin content (Test Example 3), it can be seen that a change in moisture content does not affect the content of the active ingredient in the Chrysanthemum zawadskii extract, such as Linarin.

### [Test Example 5] Evaluation of disintegration (dispersity) of finished product

In order to confirm stability of the pharmaceutical composition (600 mg, finished product) including the Chrysanthemum zawadskii extract prepared in Example 3, a finished product (600 mg) was left for 9 months at 25°C, 35°C, and 40°C, respectively, and then the disintegration (dispersity) was evaluated over time, and the result thereof was shown in Table 7 below.

**[Table 7]**

| | Month | Disintegration time | | |
|---|---|---|---|---|
| | | 25°C | 35°C | 40°C |
| Finished product, 600 mg | 0 | Within 20 minutes | Within 20 minutes | Within 20 minutes |
| | 1 | Within 20 minutes | Within 20 minutes | Within 20 minutes |
| | 2 | Within 20 minutes | Within 20 minutes | Within 20 minutes |
| | 3 | Within 20 minutes | Within 20 minutes | Within 20 minutes |
| | 4 | Within 20 minutes | Within 20 minutes | Within 20 minutes |
| | 5 | Within 20 minutes | Within 20 minutes | Within 20 minutes |
| | 6 | Within 20 minutes | Within 20 minutes | Within 20 minutes |
| | 7 | Within 20 minutes | Within 20 minutes | Within 20 minutes |
| | 8 | Within 20 minutes | Within 20 minutes | Within 20 minutes |
| | 9 | Within 20 minutes | Within 20 minutes | Within 20 minutes |

As a result of evaluating the disintegration of the finished product through the method as described above, as shown in Table 7, it was confirmed that disintegration was completed at the same level as at the time of initial measurement, that is, within 20 minutes, without being affected by the storage temperature and period, and it can be seen that this is different from a result that in the case of using a natural extract, as the moisture content decreases, the hardness increases, and the disintegration time increases.

## Claims

1. A pharmaceutical composition comprising Chrysanthemum zawadskii extract granules obtained by mixing a Chrysanthemum zawadskii extract and a dextrin-based compound and granulating the mixture.

2. The pharmaceutical composition of claim 1, wherein the dextrin-based compound is selected from the group consisting of maltodextrin, cyclodextrin, and mixtures thereof.

3. The pharmaceutical composition of claim 1, wherein the dextrin-based compound is maltodextrin.

4. The pharmaceutical composition of claim 1, wherein the content of the dextrin-based compound is 50 wt% to 70 wt% with respect to the total weight of the granules.

5. The pharmaceutical composition of claim 1, wherein 50 wt% to 70 wt% of crystalline cellulose is included with respect to the total weight of the pharmaceutical composition.

6. The pharmaceutical composition of claim 5, further comprising a pharmaceutically acceptable excipient.

7. The pharmaceutical composition of claim 6, wherein a solubilizer and a disintegrant are not included.

8. The pharmaceutical composition of claim 7, wherein the disintegration time of the pharmaceutical composition is within 30 minutes.

9. The pharmaceutical composition of claim 1, wherein the pharmaceutical composition does not include a moisture-proof coating agent and has a moisture-proof effect even without including the moisture-proof coating agent.

10. The pharmaceutical composition of claim 1, wherein the dextrin-based compound and the liquid Chrysanthemum zawadskii extract were mixed and then dried and ground to prepare the Chrysanthemum zawadskii extract granules.

11. The pharmaceutical composition of claim 6, wherein the excipient is a lubricant, and the lubricant is selected from the group consisting of silicon dioxide, magnesium stearate, and mixtures thereof.

12. The pharmaceutical composition of claim 1, wherein the pharmaceutical composition is formulated in the form selected from the group consisting of tablets, pills, powders, granules, capsules, suspensions, oral liquids, emulsions, syrups, and freeze-dried preparations.

13. The pharmaceutical composition of claim 12, wherein the pharmaceutical composition is formulated in the form of tablets.

14. The pharmaceutical composition of claim 13, wherein the tablet includes a coating layer, and the coating layer includes an aqueous coating agent.

15. The pharmaceutical composition of claim 14, wherein the aqueous coating agent comprises a cellulose-based compound selected from the group consisting of hydroxypropylmethylcellulose, methylcellulose, ethylcellulose, and hydroxypropylcellulose.

16. A food composition comprising Chrysanthemum zawadskii extract granules obtained by mixing a Chrysanthemum zawadskii extract and a dextrin-based compound and granulating the mixture.

17. The food composition of claim 16, wherein the food composition is for preventing or improving arthritis disease.
